# EUROPÄISCHE PATENTSCHRIFT

(11) **EP 1 239 905 B1**
(45) Veröffentlichungstag und Bekanntmachung des Hinweises auf die Patenterteilung: **02.12.2009**
(21) Anmeldenummer: 00990720.5
(22) Anmeldetag: 12.12.2000
(51) Int. Cl.: A61M 15/00

(54) **VORRATSSYSTEM FÜR ARZNEIMITTEL IN PULVERFORM UND DAMIT AUSGESTATTETER INHALATOR**
STORAGE SYSTEM FOR MEDICAMENTS IN POWDER FORM AND INHALER PROVIDED THEREWITH
SYSTEME DE STOCKAGE POUR MEDICAMENTS PULVERULENTS ET INHALATEUR POURVU DE CE SYSTEME DE STOCKAGE

(30) Priorität: 18.12.1999 DE 19961300
(43) Veröffentlichungstag der Anmeldung: 18.09.2002
(73) Patentinhaber: MEDA Pharma GmbH & Co. KG, 61352 Bad Homburg (DE)
(72) Erfinder: GOEDE, Joachim, 63457 Hanau (DE); LANGE, Karl-Heinz, 32257 Bünde (DE)
(86) Internationale Anmeldenummer: PCT/EP2000/012590
(87) Internationale Veröffentlichungsnummer: WO 2001/043801

(56) Entgegenhaltungen:
- WO-A-00/64519
- WO-A-01/39823
- WO-A1-01/39823
- US-A- 5 169 029
- US-A- 5 524 613
- US-A- 5 664 557
- US-A- 5 778 873
- US-A- 5 840 279

## Beschreibung

Die Erfindung betrifft ein Vorratssystem für Arzneimittel in Pulverform in Form eines Arzneimittelpulverpatronensystems und einen damit ausgestatteten Inhalator.

### Hintergrund der Erfindung

Auf dem Gebiet der Behandlung von Bronchialerkrankungen aber auch anderen Erkrankungen, bei denen eine Medikation über den Atemwegstrakt erfolgen kann, ist es bekannt, neben der Zerstäubung von Lösungen oder Suspensionen zu inhalierbaren Aerosolen pulverförmige Medikamente zu applizieren. Beispiele für solche Medikamente sind in der Literatur vielfach beschrieben, rein exemplarisch weisen wir auf WO 93/11773. EP 0 416 950 A1 und EP 0 416 951 A1 hin.

Eine gebräuchliche Applikationsform ist dabei die Zuführung über ein Inhalationsgerät (Inhalator).

Bei Inhalatoren für pulverförmige Arzneimittel sind sowohl solche zur Applikation einer Einzeldosis bekannt, als auch Inhalationsgeräte, die einen Vorrat für eine Mehrzahl von Arzneimitteldosen aufweisen. Dabei ist es bekannt, entweder getrennte Vorratsräume für jeweils eine einzelne Dosis vorzusehen oder einen einzelnen Aufnahmeraum zur Aufnahme einer Vielzahl von Dosen eines Medikaments.

Bei Inhalatoren, bei denen eine Vielzahl von Einzeldosen in separaten Vorratsräumen vorgesehen ist, sind solche bekannt, bei denen einzelne Räume des Inhalators jeweils mit einer Arzneimitteldosis befüllt sind.

Ein Beispiel eines solchen Inhalators ist in US-A 5,301,666 beschrieben. Es ist aber auch bekannt, eine Vielzahl von Arzneimittelpulverdosen jeweils getrennt in Räumen sog. Blisterverpackungen unterzubringen. Ein Beispiel für eine solche Blisterverpackung zur Verwendung mit einem Inhalator ist in DE 44 00 083 C2 beschrieben. Eine solche Blisterverpackung, die zugleich als Einweginhalator ausgebildet ist, ist beispielsweise in DE 44 00 084 A1 beschrieben.

Ein Inhalationsgerät, in das Blisterverpackungen eingelegt werden können, die jeweils getrennte Vorratsräume für einzelne Dosen eines pulverförmigen Arzneimittels enthalten und die mit Hilfe des Inhalationsgerätes nacheinander geleert werden können, ist beispielsweise in DE 195 23 516 C1 beschrieben.

Beispiele von Inhalatoren mit einem Vorratsraum für eine Vielzahl von Arzneimitteldosen sind im Stand der Technik vielfältig beschrieben. Ein Beispiel mit einem auswechselbaren Vorratsbehälter ist in DE-PS 846 770 beschrieben, ein weiteres in WO 95/31237.

Ein wesentliches Problem bei Inhalationssystemen, bei denen eine Vielzahl von Dosen eines medizinisch wirksamen Stoffes in einem gemeinsamen Vorratsraum untergebracht ist, ist die Zumessung einer einzelnen Dosis für einen einzelnen Inhalationsvorgang. Hierzu sind eine Vielzahl von Lösungsvorschlägen gemacht worden, wie beispielsweise in US-A 2,587,215 und US-A 4,274,403 beschrieben. Andere Formen von Anordnungen zum Dosieren einer einzelnen Arzneimittelpulverdosis aus einem Vorratsraum für eine Vielzahl von Arzneimitteldosen sind weiterhin in WO 92/09322, WO 93/16748 und DE 35 35 561 C2 sowie in GB 2 165 159 A beschrieben. Eine auswechselbare Patrone zur Aufnahme einer Vielzahl von Dosen eines Arzneimittelpulvers mit einem integrierten Dosierschieber ist aus DE 195 22 415 A1 oder US 5840 279 bekannt.

Ein weiteres wesentliches Problem bei der Inhalation von Arzneimittelpulver ist die Zerlegung der galenischen Pulverformulierungen in lungengängige Partikel. Die auf diesem Wege verabreichten Wirkstoffe werden in aller Regel mit Trägerstoffen zusammengeführt, um eine vernünftige Dosierbarkeit des medizinisch wirksamen Stoffes zu erreichen und um weitere Eigenschaften des Arzneimittelpulvers einzustellen, die z. B. die Lagerfähigkeit betreffen können.

Lösungsansätze für Ausbildungen bei Pulverinhalatoren, mit denen die Bereitstellung lungengängiger Partikel in einem Luftstrom zur Inhalation erreicht werden soll, sind beispielsweise in EP 0 640 354 A2, US-A 5,505,196, US-A 5,320,714, US-A 5,435,301, US-A 5,301,666, DE 195 22 416 A1 und WO 97/00703 beschrieben. Dabei sind auch Vorschläge bekannt, Hilfsenergie zur Erzeugung des Luftstromes einzusetzen, beispielsweise aus ZA-A 916741.

Ganz allgemein ist es bei der medikamentösen Behandlung von Krankheiten bekannt, verschiedene pharmakodynamisch aktive Wirkstoffe zusammen einzusetzen. Bei Medikamenten in Tabletten- oder Salbenform ist dies seit langem bekannt und üblich. Dabei ist lediglich von pharmakologischer Seite zu berücksichtigen, daß sich die Wirkstoffe oder galenischen Stoffe nicht gegenseitig nachteilig beeinflussen, sowohl hinsichtlich der Wirkung auf den Organismus als auch hinsichtlich der Resorption.

Auch bei Einsatz von Medikamenten zur Inhalation in Pulverform ist es bekannt. Wirkstoffe durch Applikation vorgefertigter Wirkstoffmischungen zu kombinieren. Entsprechende Vorschläge finden sich in EP 0 416 951 A1 und WO 93/11773 beispielsweise für die Kombination von Salmeterol und Fluticasone oder Formoterol und Budesonid. Allerdings besteht das Problem bei der Vorfertigung von Wirkstoffmischungen bei losen Pulvern darin, daß durch chemische oder physikalische Reaktionen der Wirkstoffe in den Mischungen diese insbesondere während einer längeren Lagerzeit verändert werden können, was zu Wirkungsabfall, Inhomogenitäten in der Mischung, die zu Dosisstreuungen oder unerwünschten Nebenwirkungen führen können.

Um solche Nachteile zu vermeiden, werden die pharmakodynamisch aktiven Wirkstoffe daher oft sequentiell, d.h. getrennt nacheinander appliziert. Häufig kommen dabei auch unterschiedliche Applikationssysteme für verschiedene Wirkstoffe zur Anwendung. Dies stellt nicht nur für den Anwender eine Erschwerung der Anwendung dar, die zu Verwechslungen. Anwendungsfehlern und Abfall der Compliance führen kann, sondern macht eine solche Medikamentierung bei bestimmten Krankheiten undurchführbar. Solche Krankheiten können insbesondere solche sein, deren Symptome anfallsweise auftreten und bei denen durch einen solchen Anfall die Handlungs- und Koordinationsfähigkeit des Patienten durch physische oder psychische Beeinträchtigungen beschränkt sein kann. Ein typisches Beispiel dafür sind Asthmaanfälle.

Der Erfindung liegt daher die Aufgabe zugrunde, bekannte Systeme zur Applikation pulverförmiger Arzneimittel zu verbessern.

Diese Aufgabe wird erfindungsgemäß gelöst durch ein Anzneimittel pulver patronen system nach Anspruch 1.

Mit dem System gemäß der Erfindung ist es möglich, pharmakodynamisch aktive Wirkstoffe in Form von pulverigen Arzneimitteln jeweils in getrennten Vorratsräumen für eine Vielzahl von Dosen bereitzustellen und dennoch eine zusammenhängende und zeitlich koordinierte Dosierung und Applikation der Wirkstoffe in Kombination durchzuführen, ohne daß gegenüber einer herkömmlichen Inhalation eines einheitlichen Arzneimittelpulvers zusätzliche Anforderungen an den Patienten oder den medizinischen Betreuer bestehen oder ein zusätzlicher Handhabungsaufwand erforderlich ist.

Die Erfindung eröffnet auch die Möglichkeit, durch Kombination von getrennten Vorratsräumen in Form von Teilpatronen mit dem gleichen pulverförmigen Arzneimittel eine sehr genau auf den Patienten abgestimmte Dosierung durch den Arzt vorzunehmen, ohne daß eine entsprechende Vielzahl von verschiedenen Patronen mit unterschiedlichen Dosiereinrichtungen erforderlich wären.

Für eine besonders gute Kombinationswirkung der Wirkstoffe ist es vorteilhaft, wenn das Vorratssystem ferner eine Einrichtung zur annähernd zeitgleichen Applikation von Arzneimitteldosen aus wenigstens zwei der voneinander getrennten Vorratsräumen aufweist.

In einer vorteilhaften Ausführungsform weist das Arzneimittelpulverpatronensystem eine Arzneimittelpulverpatrone auf, die wenigstens zwei Vorratskammern aufweist. Dabei ist es zweckmäßig, wenn die Arzneimittelpulverpatrone eine Dosiereinrichtung aufweist, wobei die Dosiereinrichtung für jede der Vorratskammern eine Dosierkammer zur Zumessung einer vorbestimmten Menge eines jeden in den Vorratskammern vorgesehenen medizinisch wirksamen Stoffes hat.

Für eine freiere Kombination der Wirkstoffe oder Dosismengen durch einen Arzt oder Apotheker kann es aber auch zweckmäßig sein, wenn das das Vorratssystem ein Arzneimittelpulverpatronensystem mit wenigstens zwei Arzneimittelpulverpatronen umfaßt, die jeweils wenigstens eine Vorratskammer aufweisen.

Dabei ist es besonders vorteilhaft, wenn jede Arzneimittelpulverpatrone eine Dosiereinrichtung aufweist, wobei jede Dosiereinrichtung für jede der Vorratskammern eine Dosierkammer zur Zumessung einer vorbestimmten Menge eines jeden in den Vorratskammern der Arzneimittelpulverpatronen vorgesehenen medizinisch wirksamen Stoffes hat.

Insbesondere, wenn in einem Inhalator das Arzneimittelpulverpatronensystem mit verschiedenen Wirkstoffkombinationen nacheinander verwendet werden soll, ist es zur Vermeidung von Wechselwirkungen der pulverförmigen Medikamente zweckmäßig, wenn das Arzneimittelpulverpatronensystem eine Einrichtung zum Zusammenführen der mit den Dosierkammern zugemessenen Stoffmengen aufweist.

In einer vorteilhaften Ausführungsform sind die Dosiereinrichtungen koppelbar.

Für besonders gute Handhabung ist es vorteilhaft. wenn die Arzneimittelpulverpatronen mechanisch koppelbar sind.

Eine besonders große Sicherheit vor Fehlmedikation läßt sich erhalten, wenn die Arzneimittelpulverpatronen ferner an Wirkflächen, die zum Koppeln der Arzneimittelpulverpatronen dienen, Kodiermittel aufweisen, die ein Koppeln nur von dafür bestimmten Arzneimittelpulverpatronen erlauben. Dazu ist es zweckmäßig, wenn die Kodiermittel Erhöhungen und Vertiefungen aufweisen, die ein Koppeln nur von dafür bestimmten Arzneimittelpulverpatronen mit aufeinander abgestimmten Erhöhungen und Vertiefungen erlauben, insbesondere, wenn die Erhöhungen und Vertiefungen in Form einer Matrix angeordnet sind.

Für eine Zusammenstellung von Patronen für das erfindungsgemäße Arzneimittelpulverpatronensystem z.B. durch einen Apotheker ist es günstig, wenn die Arzneimittelpulverpatronen ferner an ihren Wirkflächen, die zum Koppeln der Arzneimittelpulverpatronen dienen, Rastmittel aufweisen, die ein Koppeln von dafür bestimmten Arzneimittelpulverpatronen erlauben. Eine besonders hohe Sicherheit gegen Manipulationen durch Nichtfachleute oder zur Verhinderung einer Wiederbefüllung der Arzneimittelpulverpatronen läßt sich erhalten, wenn die Rastmittel so ausgebildet sind, daß die miteinander gekoppelten Arzneimittelpulverpatronen nicht ohne Beschädigungen wieder voneinander getrennt werden können.

Für die Handhabung durch den Patienten ist es vorteilhaft, wenn das Arzneimittelpulverpatronensystem ferner eine Einrichtung zur Anzeige der in den Vorratskammern verbliebenen oder aus den Vorratskammern entnommenen Menge an Arzneimitteldosen aufweist, insbesondere, wenn die Arzneimittelpulverpatronen miteinander und mit der Einrichtung zur Anzeige der in den Vorratskammern verbliebenen oder aus den Vorratskammern entnommenen Menge an Arzneimitteldosen koppelbar sind und/oder das Arzneimittelpulverpatronensystem für jede der Vorratskammern eine Einrichtung zur Anzeige der in den Vorratskammern verbliebenen oder aus den Vorratskammern entnommenen Menge an Arzneimitteldosen aufweist.

Fertigungstechnisch besonders günstig ist es, wenn das Arzneimittelpulverpatronensystem zwei oder mehr ohne die Rast- und Kodiermittel gleiche, spiegelbildlich gleiche oder punktsymmetrisch gleiche Arzneimittelpulverpatronen umfaßt.

Für eine besonders vielfältige Anwendung ist es vorteilhaft, wenn die Dosiereinrichtungen der einzelnen Arzneimittelpulverpatronen Dosierkavitäten mit gleichem oder unterschiedlichem Volumen aufweisen.

Die Erfindung läßt sich wirtschaftlich besonders gut nutzen bei einem Inhalator für pulverförmige Arzneimittel mit einem solchen Arzneimittelpulverpatronensystem.

### Anwendung der Erfindung

Mit dem Arzneimittelpulverpatronensystem gemäß der Erfindung ist es möglich, pharmakodynamisch aktive Wirkstoffe in Form von pulverigen Arzneimitteln jeweils in getrennten Vorratsräumen für eine Vielzahl von Dosen bereitzustellen und dennoch eine zusammenhängende und zeitlich koordinierte Dosierung und Applikation der Wirkstoffe in Kombination durchzuführen, ohne daß gegenüber einer herkömmlichen Inhalation eines einheitlichen Arzneimittelpulvers zusätzliche Anforderungen an den Patienten oder den medizinischen Betreuer bestehen oder ein zusätzlicher Handhabungsaufwand erforderlich ist.

Weiterhin ist es möglich, pulverförmige Arzneimittel in Wirkstoffkombinationen zur einheitlichen Inhalation bereitzustellen. deren einzelne Wirkstoffe untereinander Unverträglichkeiten hinsichtlich der Lagerfähigkeit, ihrer Beständigkeit oder ihrer Dosierbarkeit aufweisen.
Beispiele für solche Wirkstoffkombinationen können sein, ohne daß die Aufzählung beschränkend wäre: Budesonid kombiniert mit Formoterol, Fluticason kombiniert mit Formoterol, Fluticason kombiniert mit Salmeterol, Fenoterol kombiniert mit Ipatropiumbromid, Glycopyrrolate kombiniert mit Formoterol, Loteprednol kombiniert mit Formoterol, Loteprednol kombiniert mit Glycopyrrolate, Mometason mit Formoterol, Mometason kombiniert mit Salmeterol, DNCG (Dinatriumcromoglycat) mit Reproterol.

Elemente für Wirkstoffkombinationen, für die die Erfindung einsetzbar ist, können ferner beispielsweise sein aus der Gruppe der Betasympatikomimetika: Salbutamol, Reproterol, Fenoterol, Formoterol, Salmeterol. Beispiele aus der Gruppe der Kortikosteroide können sein: Budesonid, Beclomethason, Fluticason, Triamcinolon, Loteprednol. Mometason, Flunisolid, Ciclosonid. Beispiele aus der Gruppe der Anticholinergica können sein: Ipatropiumbromid, Tiotropiumbromid, Glycopyrrolate.

Beispiele aus der Gruppe der Analgetica und Migränetherapeutika können sein: Morphin, Tramadol, Flupirtin, Sumatryptan. Aus der Gruppe der Peptide und Proteine können beispielsweise verwendet werden: Cetrorelix, Insulin, Calcitonin, Parathyroid Hormon, Faktor VIII Analoge, Alpha Interferon, Beta Interferon, Heparin, FSH (Follikel Stimulierendes Hormon). Collistin, Tobramycin.

Die Anwendung ist nicht auf die hier genannten Wirkstoffe beschränkt. Das beschriebene Arzneimittelpulverpatronensystem eignet sich für alle Wirkstoffe, die in Pulverform dosiert und inhalativ appliziert werden können. Durch entsprechende Modifikation des Systems und der Dosiervorrichtung eignet sich die beschriebene Erfindung auch zur Kombination von Wirkstoffen, die flüssige Zubereitungen, zum Beispiel Lösungen oder Suspensionen von pharmakodynamisch aktiven Wirkstoffen, enthalten.

Arzneimittelpulverformulierungen, die sinnvoll mit dem Arzneimittelpulverpatronensystem gemäß der Erfindung verwendet werden können, können verschiedene Wirkstoffe enthalten, wie beispielsweise Analgetika, Antiallergika, Antibiotika, Anticholinergika, Antihistaminika, antiinflammatorisch wirkende Substanzen, Antipyretika, Kortikoide, Steroide, Antitussiva, Bronchodilatatoren, Diuretika, Enzyme, Herz-Kreislauf wirksame Substanzen, Hormone, Proteine und Peptide. Beispiele für Analgetika sind Codein, Diamorphin, Dihydromorphin, Ergotamin, Fentanyl und Morphin: Beispiele für Antiallergika sind Cromoglicinsäure und Nedocromil; Beispiele für Antibiotika sind Cephalosporine, Fusafungin, Neomycin, Penicilline, Pentamidin, Streptomycin, Sulfonamide und Tetracycline, Collistin, Tobramycin; Beispiele für Anticholinergika sind Atropin, Atropinmethonitrat, Ipratropiumbromid, Oxitropiumbromid, Trospiumchlorid und Tiotropiumbromid; Beispiele für Antihistaminika sind Azelastin, Flezelastin und Methapyrilen: Beispiele für antiinflammatorisch wirksame Substanzen sind Beclometason, Budesonid, Loteprednol, Dexamethason, Flunisolid, Fluticason, Tipredane, Triamcinolon, Mometason; Beispiele für Antitussiva sind Narcotin und Noscapin; Beispiele für Bronchodilatatoren sind Bambuterol. Bitolterol, Carbuterol. Clenbuterol, Ephedrin, Epinephrin, Formoterol, Fenoterol, Hexoprenalin, Ibuterol, Isoprenalin, Isoproterenol, Metaproterenol, Orciprenalin, Phenylephrin, Phenylpropanolamin, Pirbuterol, Procaterol, Reproterol, Rimiterol, Salbutamol, Salmeterol, Sulfonterol, Terbutalin und Tolobuterol; Beispiele für Diuretika sind Amilorid und Furosemid; ein Beispiel für Enzyme ist Trypsin; Beispiele für Herz-Kreislauf wirksame Substanzen sind Diltiazem und Nitroglycerin: Beispiele für Hormone sind Cortison, Hydrocortison und Prednisolon; Beispiele für Proteine und Peptide sind Cyclosporine, Cetrorelix, Glucagon und Insulin. Weitere Wirkstoffe, die eingesetzt werden können, sind Adrenochrom, Colchicin, Heparin, Scopolamin. Die beispielhaft angeführten Wirkstoffe können als freie Basen oder Säuren oder als pharmazeutisch verträgliche Salze eingesetzt werden. Als Gegenionen können beispielsweise physiologisch verträgliche Erdalkali- oder Alkalimetalle oder Amine sowie beispielsweise Acetat, Benzolsulfonat, Benzoat, Hydrogencarbonat, Hydrogenartrat, Bromid, Chlorid, Iodid, Carbonat, Citrat, Fumarat, Malat, Maleat, Cluconat, Lactat, Pamoat und Sulphat eingesetzt werden. Es können auch Ester eingesetzt werden, beispielsweise Acetat, Acetonid, Propionat, Dipropionat, Valerat.

Die Erfindung ermöglicht aber nicht nur die kombinierte Applikation von verschiedenen pulverförmigen Arzneimitteln in jeweils gleichen oder unterschiedlichen Teildosen, sondern eröffnet auch die Möglichkeit, durch Kombination von Teilpatronen mit dem gleichen pulverförmigen Arzneimittel eine sehr genau auf den Patienten abgestimmte Dosierung durch den Arzt vorzunehmen, ohne daß eine entsprechende Vielzahl von verschiedenen Patronen mit unterschiedlichen Dosiereinrichtungen erforderlich wäre, z.B. im Vergleich zu der aus WO 97/00703 bekannten Patrone.

Werden die Teilpatronen z.B. in einer Stufung von Dosiervolumina von 1. 3 und 5 ml geführt, lassen sich damit bei Verwendung auch nur einer Teilpatrone oder Ersatz der zweiten Patrone durch einen Dummy als Schutz Dosisvolumina von 1, 2, 3, 4, 5, 6, 8, 10 ml erhalten.

Die Erfindung soll im folgenden anhand von Ausführungsbeispielen näher erläutert werden. Es zeigen:
- Figur 1: ein erfindungsgemäßes Arzneimittelpulverpatronensystem in perspektivischer Ansicht;
- Figur 2: einen Längsschnitt durch ein erfindungsgemäßes Arzneimittelpulverpatronensystem;
- Figur 3: einen Querschnitt durch das erfindungsgemäßes Arzneimittelpulverpatronensystem entlang der Linie A-A in Figur 2;
- Figur 4: einen Längsschnitt durch eine Arzneimittelpatrone des erfindungsgemäßen Arzneimittelpulverpatronensystems entlang der Linie B-B in Figur 3;
- Figur 5: einen Längsschnitt durch eine Arzneimittelpatrone des erfindungsgemäßen Arzneimittelpulverpatronensystems wie in Figur 2 (Sekundärpatrone);
- Figur 6: einen Längsschnitt durch eine andere Arzneimittelpatrone des erfindungsgemäßen Arzneimittelpulverpatronensystems wie in Figur 2 (Primärpatrone);
- Figur 7: eine Ansicht einer Arzneimittelpatrone des erfindungsgemäßen Arzneimittelpulverpatronensystems (Rückseite zur Darstellung in Figur 2) (Primärpatrone);
- Figur 8: eine Ansicht einer Arzneimittelpatrone des erfindungsgemäßen Arzneimittelpulverpatronensystems ähnlich wie in Figur 2 (Sekundärpatrone); und
- Figur 9: eine Seitenansicht der Arzneimittelpatrone des erfindungsgemäßen Arzneimittelpulverpatronensystems (Primärpatrone).

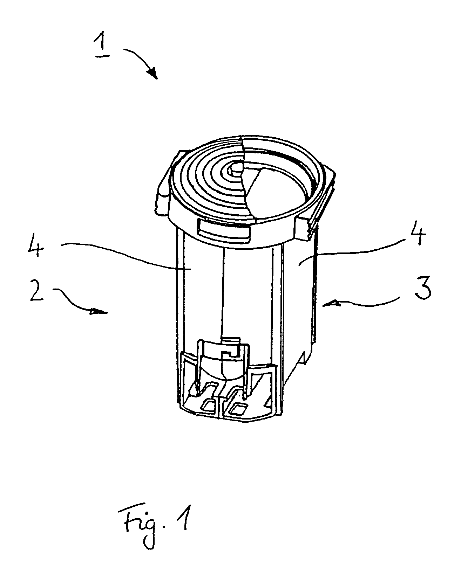

Fig. 1 zeigt eine perspektivische Ansicht eines erfindungsgemäßen Vorratsystems, hier in Form eines Arzneimittelpulverpatronensystems 1 zum austauschbaren Einsetzen in einen Pulverinhalator. Das dargestellte Arzneimittelpulverpatronensystem 1 besteht hier aus zwei Arzneimittelpulverpatronen 2 und 3 mit jeweils einer Vorratskammer 4 für ein Arzneimittelpulver.

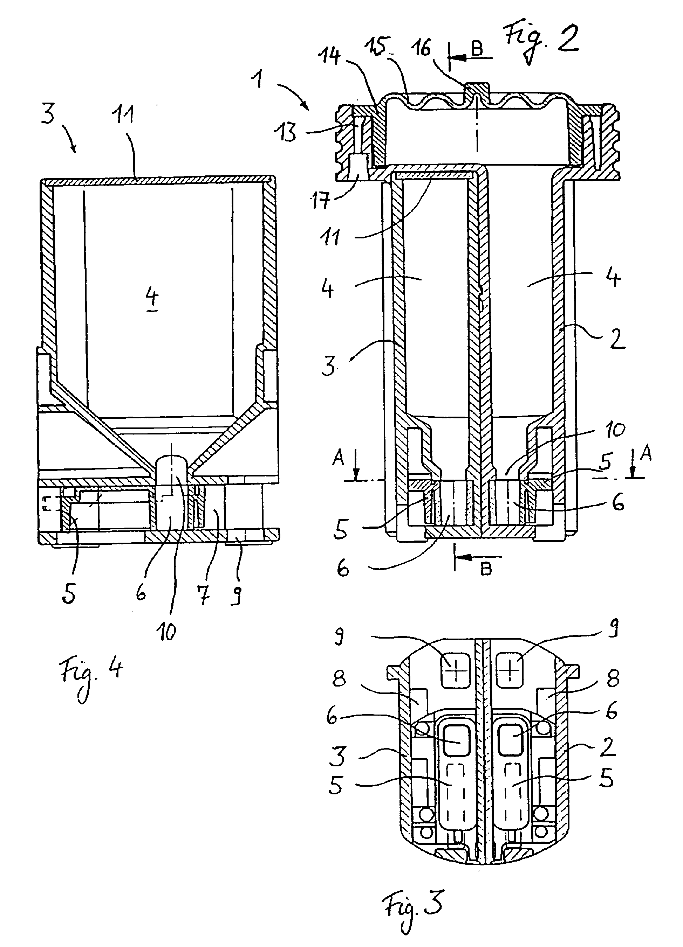

In Fig. 2 ist ein Längsschnitt durch das Arzneimittelpulverpatronensystem 1 dargestellt. Auch hier ist gut erkennbar, daß das Arzneimittelpulverpatronensystem 1 in diesem Falle aus zwei Teilpatronen 2 und 3 besteht. Der Übersichtlichkeit halber wird im folgenden unterschieden zwischen der Primärpatrone 2 und der Sekundärpatrone 3. Jede der Arzneimittelpulverpatronen 2 und 3 umfaßt eine Vorratskammer 4 zur Aufnahme eines gleichen oder verschiedener Arzneimittelpulver, wie bereits zuvor erläutert.

Weiterhin weist jede der Arzneimittelpulverpatronen 2 und 3 jeweils eine Dosiereinrichtung in Form eines Dosierschiebers 5 auf. Jeder der Dosierschieber 5 umfaßt eine Dosierkavität 6, deren Aufnahmevolumen die für eine Inhalation bereitzustellende Dosiermenge bildet.

Wie in Fig. 3, einem Querschnitt durch das Arzneimittelpulverpatronensystem 1 entlang der Linie A-A in Fig. 2 in Höhe der Dosierschieber 5, zu erkennen ist, sind die Dosierschieber 5 in einem Dosierschieberkanal 7 zumindest aus der dargestellten Füllstellung in eine Entleerungsstellung verschiebbar. Die Dosierschieber 5 der Primärpatrone 2 und der Sekundärpatrone 3 sind hier der Einfachheit halber als zueinander spiegelbildlich und mit gleich großen Dosierkavitäten 6 dargestellt.

In der in Fig. 3 dargestellten Befüllungsstellung kann, wie in Fig. 2 gut zu erkennen ist, Arzneimittelpulver aus den Vorratskammern 4 in die Dosierkavitäten 6 fallen. Wenn die Dosierkavitäten 6 mit einem Arzneimittelpulver wie gewünscht gefüllt sind, können die Dosierschieber 5 durch nicht dargestellte Eingriffsmittel eines Pulverinhalators, wie z.B. in US-A-5,840,279 beschrieben, in eine nicht dargestellte Entleerungsposition verschoben werden. Die Eingriffsmittel des Pulverinhalators ragen dabei zweckmäßig durch entsprechend in den unteren Bereichen der Primärpatrone 2 und der Sekundärpatrone 3 vorgesehene Aussparungen 8.

Die Entleerungsstellung ist dann erreicht, wenn die Dosierkavitäten 6 sich über den Entleerungsöffnungen 9 befinden. Wenn die Dosierschieber 5 diese Stellung erreicht haben, kann das Arzneimittelpulver aus den Dosierkavitäten 6 durch die Entleerungsöffnungen 9 z.B. in einen nicht dargestellten Pulverkanal eines Inhalators fallen.

Werden unterschiedliche Arzneimittelpulver in den Vorratskammern 4 der Primärpatrone 2 und der Sekundärpatrone 3 untergebracht, vermengen sich die Arzneimittelpulver dann entsprechend in dem Pulverkanal des Inhalators, möglichst unterstützt durch den Inhalationsluftstrom.

In Fig. 4 ist ein Längsschnitt durch die Sekundärpatrone 3 entlang der Linie B-B in Fig. 2 dargestellt. Dort ist gut die Befüllungsstellung des Dosierschiebers 5 erkennbar mit der Dosierkavität 6 unter dem Loch 10 der Unterseite der Vorratskammer 4. Zum Erreichen der Entleerungsstellung wird der Dosierschieber 5 in Fig. 4 so weit nach rechts verschoben, bis sich diese Dosierkavität 6 mit der Entleerungsöffnung 9 deckt und das Arzneimittelpulver nach unten herausfallen kann.

Die Oberseite der Vorratskammer 4 der Sekundärpatrone 3 wird nach Befüllung der Sekundärpatrone 3 mit einem Arzneimittel durch beispielsweise einen stabilen Foliendeckel 11 verschlossen, z.B. durch Ultraschallschweißen oder in anderer geeigneter Form.

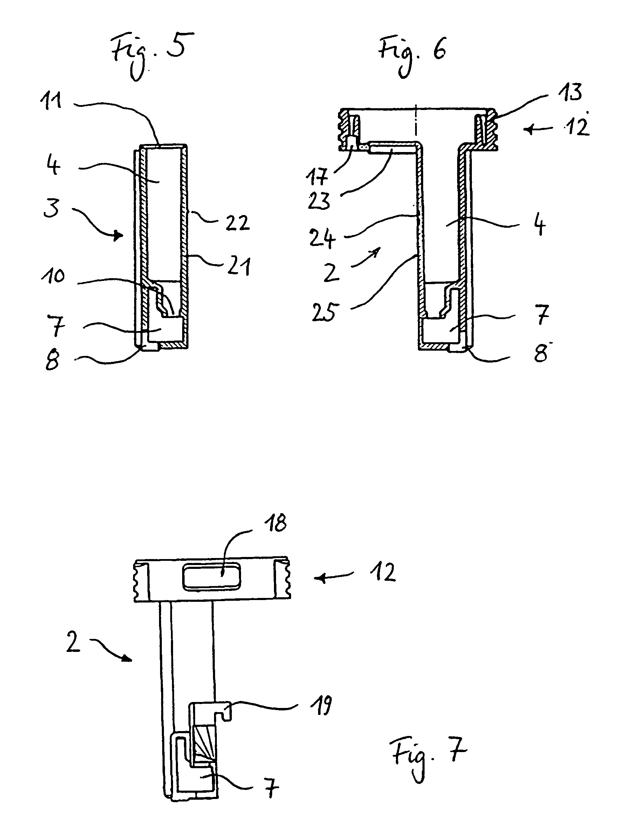

In Fig. 5 ist die Sekundärpatrone 3 in gleicher Weise wie in Fig. 2 dargestellt, jedoch ohne den Dosierschieber 5 und die Primärpatrone 2.

Entsprechend ist in Fig. 6 die Primärpatrone 2 dargestellt. Wie in Fig. 2 zu sehen ist, ist in gleicher Weise wie bei der Sekundärpatrone 3 auf der Unterseite der Primärpatrone 2 ein Dosierschieber angeordnet. Die Wirkungsweise und die Betätigung des Dosierschiebers entspricht derjenigen des Dosierschiebers der Sekundärpatrone 3, wie oben beschrieben.

Davon abweichend hat die Primärpatrone 2 einen oberen Bereich 12, der im zusammengesetzten Zustand der beiden Patronen 2 und 3 auch die Sekundärpatrone 3 mit abdeckt, wie gut in Fig. 2 zu sehen.

Bei dem dargestellten Ausführungsbeispiel ist nämlich in dem oberen Bereich 12 in einem Ringkanal 13 zugleich eine Einrichtung zur Anzeige der in den Vorratskammern 4 verbliebenen oder aus den Vorratskammern 4 entnommenen Menge an Arzneimitteldosen vorgesehen (jedoch nicht im einzelnen dargestellt).

Diese Anzeige kann z.B. als Folienstreifen mit entsprechenden Markierungen ausgebildet sein, wie im einzelnen in WO 97/00703 beschrieben. Entsprechend ist die Vorratskammer 4 in dem oberen Bereich 12 der Primärpatrone 2 durch einen Deckel 14 verschlossen, der zugleich auch den Ringkanal 13 für den Folienstreifen zur Anzeige der Arzneimitteldosen abdeckt. Die Oberseite des Deckels 14 ist dabei als Wellfeder 15 ausgebildet. um den Vorratsraum nicht nur elastisch zu verschließen, sondern auch über den zentralen Stift 16 einen Längenausgleich und einen weitgehend spielfreien Sitz des Arzneimittelpulverpatronensystems 1 in einem geeigneten Inhalator sicherzustellen. Ein solcher Inhalator ist annäherungweise ebenfalls in WO 97/00703 beschrieben.

Im oberen Bereich 12 der Primärpatrone 2 ist in Verbindung mit dem Ringkanal 13 noch eine Ausnehmung 17 vorgesehen zum Eingriff eines Antriebsmittels eines Inhalators zum Antrieb des Anzeigeelementes für die Zahl der verbliebenen bzw. entnommenen Arzneimitteldosen.

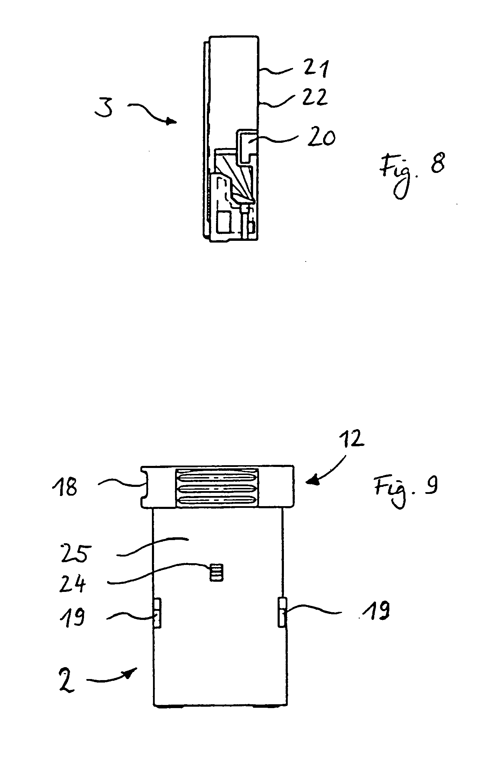

Weiterhin ist zweckmäßig im oberen Bereich 12 in Höhe des Ringkanals 13 in der Primärpatrone 12 ein Sichtfenster 18 vorgesehen, das das Ablesen der Einrichtung zur Anzeige der entnommenen oder verbliebenen Arzneimitteldosen ermöglicht (Fig. 7). In Fig. 7 ist ebenfalls gut zu erkennen, daß die Primärpatrone 2 mit Hakenelementen 19 versehen ist, die mit entsprechenden Vertiefungen 20 in der Sekundärpatrone 3 zusammenwirken können (Fig. 8). Weiterhin ist in der der Primärpatrone 2 zugewandten Wandung 21 der Sekundärpatrone 3 ein keilförmiger Vorsprung 22 vorgesehen, der beim Einschieben der Sekundärpatrone 3 von unten in eine Aussparung 23 im oberen Bereich 12 der Primärpatrone 2 zu einer elastischen Verformung der Wandung 21 führt und mit entsprechend ausgebildeter Vertiefung 24 in der der Sekundärpatrone 3 zugewandten Wandung 25 der Primärpatrone 2 verrastet, sobald die Sekundärpatrone 2 ihren vorgesehenen Sitz erreicht hat. Durch die Vertiefung 24 und das Zusammenwirken mit den Hakenelementen 19 der Primärpatrone 2 wird verhindert, daß die Sekundärpatrone 3 sich nach Montage seitlich von der Primärpatrone 2 weg bewegen kann. Durch die Verrastung des keilförmigen Vorsprungs 22 mit der zugehörigen Vertiefung 24 in der Primärpatrone 2 ist sichergestellt, daß die Sekundärpatrone nicht mehr nach unten gezogen werden kann, um die Hakenelemente 19 und die Vertiefung 24 außer Eingriff zu bringen (Fig. 9). Nach einer solchen Montage können die Primärpatrone 2 und die Sekundärpatrone 3 nur noch durch Zerstörung voneinander getrennt werden.

Eine geeignete Kodierung kann noch auf den einander zugewandten Wandungen 25 und 21 vorgesehen werden. Dies kann erfolgen z.B. in Form kodierter Vertiefungen und Vorsprünge nach Art eines Schlüssels. Bei einer anderen Anordnung zur Vermeidung des Trennens der Patrone nach Montage kann auch eine entsprechend kodierte Matrix mit stiftförmigen Vorsprüngen und Löchern eingesetzt werden.

Es ist ohne weiteres ersichtlich, daß auch andere Ausführungsformen des erfindungsgemäßen Arzneimittelpulverpatronensystems denkbar sind, z.B. zwei symmetrische oder asymmetrische Patronen, die separate Anzeigen für die entnommenen bzw. noch vorhandenen Dosen enthalten, oder symmetrische oder asymmetrische Patronen, die zusammen mit einem Kopfelement montiert werden, das eine entsprechende Anzeige enthält.

### BEZUGSZEICHENLISTE

- 1: Arzneimittelpulverpatronensystem
- 2: Primärpatrone
- 3: Sekundärpatrone
- 4: Vorratskammer
- 5: Dosierschieber
- 6: Dosierkavität
- 7: Dosierschieberkanal
- 8: Aussparung
- 9: Entleerungsöffnung
- 10: Loch
- 11: Foliendeckel
- 12: oberer Bereich von 2
- 13: Ringkanal
- 14: Deckel
- 15: Wellfeder
- 16: Stift
- 17: Ausnehmung in 13
- 18: Sichtfenster
- 19: Hakenelement
- 20: Vertiefung
- 21: Wandung
- 22: keilförmiger Vorsprung
- 23: Aussparung von 12
- 24: Vertiefung
- 25: Wandung

## Patentansprüche

1. Arzneimittelpulverpatronensystem (1) zur Verwendung in einer medizinischen Applikationseinrichtung, zur Aufnahme einer Vielzahl von Dosen wenigstens eines medizinisch wirksamen Stoffes, und zum austauschbaren Einsetzen in einen Pulverinhalator, mit wenigstens zwei voneinander getrennten Vorratsräumen in Form von vonratskammern (4) für jeweils eine Vielzahl von Dosen eines medizinisch wirksamen Stoffes, wobei das Arzneimittelpulver patronen system zumindest eine Arzneimittelpulverpatrone umfasst, die eine Dosiereinrichtung aufweist und die Dosiereinrichtung für jede der Vorratskammem (4) eine Dosierkavität (6) zur Zumessung einer vorbestimmten Menge eines jeden in den Vorratskammern (4) vorgesehenen medizinisch wirksamen Stoffes hat.

2. Arzneimittelpulverpatronensystem (1) nach Anspruch 1,
**dadurch gekennzeichnet, dass**
das Arzneimittelpulverpatronensystem (1) ferner eine Einrichtung zur annähernd zeitgleichen Applikation von Arzneimitteldosen aus wenigstens zwei der voneinander getrennten Vorratsräumen aufweist.

3. Arzneimittelpulverpatronensystem nach Anspruch 1 oder 2,
**dadurch gekennzeichnet, dass**
das Arzneimittelpulverpatronensystem eine Arzneimittelpulverpatrone umfaßt, die wenigstens zwei Vorratskammern (4) aufweist.

4. Arzneimittelpulverpatronensystem nach Anspruch 3,
**dadurch gekennzeichnet, dass**
das Arzneimittelpulverpatronensystem eine Einrichtung zum Zusammenführen der mit den Dosierkammern zugemessenen Stoffmengen aufweist.

5. Arzneimittelpulverpatronensystem nach einem der Ansprüche 1 oder 2,
**dadurch gekennzeichnet, dass**
das Arzneimittelpulverpatronensystem (1) wenigstens zwei Arzneimittelpulverpatronen (2, 3) umfaßt, die jeweils wenigstens eine Vorratskammer (4) aufweisen.

6. Arzneimittelpulverpatronensystem nach Anspruch 5,
**dadurch gekennzeichnet, dass**
jede Arzneimittelpulverpatrone (2, 3) eine Dosiereinrichtung (5) aufweist, wobei jede Dosiereinrichtung (5) für jede der Vorratskammern (4) eine Dosierkavität (6) zur Zumessung einer vorbestimmten Menge eines jeden in den Vorratskammern (4) der Arzneimittelpulverpatronen (2, 3) vorgesehenen medizinisch wirksamen Stoffes hat.

7. Arzneimittelpulverpatronensystem nach Anspruch 6,
**dadurch gekennzeichnet, dass**
das Arzneimittelpulverpatronensystem (1) eine Einrichtung zum Zusammenführen der mit den Dosierkammern zugemessenen Stoffmengen aufweist.

8. Arzneimittelpulverpatronensystem nach einem der Ansprüche 6 bis 7,
**dadurch gekennzeichnet, dass**
die Dosiereinrichtungen (5) koppelbar sind.

9. Arzneimittelpulverpatronensystem nach einem der Ansprüche 6 bis 8,
**dadurch gekennzeichnet, dass**
die Arzneimittelpulverpatronen (2, 3) mechanisch koppelbar sind.

10. Arzneimittelpulverpatronensystem nach Anspruch 9,
**dadurch gekennzeichnet, dass**
die Arzneimittelpulverpatronen (2, 3) ferner an Wirkflächen (21, 25), die zum Koppeln der Arzneimittelpulverpatronen (2, 3) dienen, Kodiermittel aufweisen, die ein Koppeln nur von dafür bestimmten Arzneimittelpulverpatronen (2, 3) erlauben.

11. Arzneimittelpulverpatronensystem nach Anspruch 10,
**dadurch gekennzeichnet, dass**
die Kodiermittel Erhöhungen und Vertiefungen aufweisen, die ein Koppeln nur von dafür bestimmten Arzneimittelpulverpatronen (2, 3) mit aufeinander abgestimmten Erhöhungen und Vertiefungen erlauben.

12. Arzneimittelpulverpatronensystem nach Anspruch 11,
**dadurch gekennzeichnet, dass**
die Erhöhungen und Vertiefungen in Form einer Matrix angeordnet sind.

13. Arzneimittelpulverpatronensystem nach einem der Ansprüche 5 bis 12,
**dadurch gekennzeichnet, dass**
die Arzneimittelpulverpatronen (2, 3) ferner an ihren Wirkflächen (21, 25), die zum Koppeln der Arzneimittelpulverpatronen (2, 3) dienen, Rastmittel (22, 24, 19, 20, 23) aufweisen, die ein Koppeln von dafür bestimmten Arzneimittelpulverpatronen (2, 3) erlauben.

14. Arzneimittelpulverpatronensystem nach Anspruch 13,
**dadurch gekennzeichnet, dass**
die Rastmittel (22, 24) so ausgebildet sind, dass die miteinander gekoppelten Arzneimittelpulverpatronen (2, 3) nicht ohne Beschädigungen wieder voneinander getrennt werden können.

15. Arzneimittelpulverpatronensystem nach einem der Ansprüche 5 bis 14,
**dadurch gekennzeichnet, dass**
das Arzneimittelpulverpatronensystem (1) ferner eine Einrichtung zur Anzeige der in den Vorratskammern (4) verbliebenen oder aus den Vorratskammern (4) entnommenen Menge an Arzneimitteldosen aufweist.

16. Arzneimittelpulverpatronensystem nach Anspruch 15,
**dadurch gekennzeichnet, dass**
die Arzneimittelpulverpatronen (2, 3) miteinander und mit der Einrichtung zur Anzeige der in den Vorratskammern (4) verbliebenen oder aus den Vorratskammern (4) entnommenen Menge an Arzneimitteldosen koppelbar sind.

17. Arzneimittelpulverpatronensystem nach einem der Ansprüche 15 oder 16,
**dadurch gekennzeichnet, dass**
das Arzneimittelpulverpatronensystem (1) für jede der Vorratskammern (4) eine Einrichtung zur Anzeige der in den Vorratskammern (4) verbliebenen oder aus den Vorratskammern (4) entnommenen Menge an Arzneimitteldosen aufweist.

18. Arzneimittelpulverpatronensystem nach einem der Ansprüche 1 bis 17,
**dadurch gekennzeichnet, dass**
das Arzneimittelpulverpatronensystem (1) zwei oder mehr ohne die Rast- und Kodiermittel (19, 20, 22, 23, 24) gleiche, spiegelbildlich gleiche oder punktsymmetrisch gleiche Arzneimittelpulverpatronen (2, 3) umfaßt.

19. Arzneimittelpulverpatronensystem nach einem der vorhergehenden Ansprüche,
**dadurch gekennzeichnet, dass**
die Dosiereinrichtungen (5) der einzelnen Arzneimittelpulverpatronen (2, 3) Dosierkavitäten (6) mit gleichem oder unterschiedlichem Volumen aufweisen.

20. Inhalator für pulverförmige Arzneimittel,
**dadurch gekennzeichnet, dass**
der Inhalator ein Arzneimittelpulverpatronensystem (1) nach einem der vorhergehenden Ansprüche umfaßt.

## Claims

1. Pharmaceutical powder cartridge system (1) for use in a medical administration device (inhaler), for receiving a multiplicity of doses of at least one medically active substance, and for exchangeable insertion into a powder inhaler, with at least two storage spaces in the form of storage chambers (4) which are separate from each other and are each intended to hold a multiplicity of doses of a medically active substance, the pharmaceutical powder cartridge system comprising at least one pharmaceutical powder cartridge, which has a metering device, and the metering device having, for each of the storage chambers (4), a metering cavity (6) for apportioning a predetermined quantity of each medically active substance provided in the storage chambers (4).

2. Pharmaceutical powder cartridge system (1) according to Claim 1, **characterized in that** the pharmaceutical powder cartridge system (1) further comprises a device for approximately simultaneous administration of pharmaceutical doses from at least two of the separate storage spaces.

3. Pharmaceutical powder cartridge system according to Claim 1 or 2, **characterized in that** the pharmaceutical powder cartridge system comprises a pharmaceutical powder cartridge which has at least two storage chambers (4).

4. Pharmaceutical powder cartridge system according to Claim 3, **characterized in that** the pharmaceutical powder cartridge system has a device for bringing together the quantities of substance apportioned by the metering chambers.

5. Pharmaceutical powder cartridge system according to one of Claims 1 or 2, **characterized in that** the pharmaceutical powder cartridge system (1) comprises at least two pharmaceutical powder cartridges (2, 3) which each have at least one storage chamber (4).

6. Pharmaceutical powder cartridge system according to Claim 5, **characterized in that** each pharmaceutical powder cartridge (2, 3) has a metering device (5), and each metering device (5) has, for each of the storage chambers (4), a metering cavity (6) for apportioning a predetermined quantity of each medically active substance provided in the storage chambers (4) of the pharmaceutical powder cartridges (2, 3).

7. Pharmaceutical powder cartridge system according to Claim 6, **characterized in that** the pharmaceutical powder cartridge system (1) has a device for bringing together the quantities of substance apportioned by the metering chambers.

8. Pharmaceutical powder cartridge system according to one of Claims 6 to 7, **characterized in that** the metering devices (5) can be coupled together.

9. Pharmaceutical powder cartridge system according to one of Claims 6 to 8, **characterized in that** the pharmaceutical powder cartridges (2, 3) can be coupled together mechanically.

10. Pharmaceutical powder cartridge system according to Claim 9, **characterized in that**, on active surfaces (21, 25) serving to couple the pharmaceutical powder cartridges (2, 3) together, the pharmaceutical powder cartridges (2, 3) also have indexing means which permit coupling only of pharmaceutical powder cartridges (2, 3) intended for this purpose.

11. Pharmaceutical powder cartridge system according to Claim 10, **characterized in that** the indexing means have elevations and depressions which permit coupling only of pharmaceutical powder cartridges (2, 3) which are intended for this purpose and which have matching elevations and depressions.

12. Pharmaceutical powder cartridge system according to Claim 11, **characterized in that** the elevations and depressions are arranged in the form of a matrix.

13. Pharmaceutical powder cartridge system according to one of Claims 5 to 12, **characterized in that**, on their active surfaces (21, 25) serving to couple the pharmaceutical powder cartridges (2, 3) together, the pharmaceutical powder cartridges (2, 3) also have locking means (22, 24, 19, 20, 23) which permit coupling of pharmaceutical powder cartridges (2, 3) intended for this purpose.

14. Pharmaceutical powder cartridge system according to Claim 13, **characterized in that** the locking means (22, 24) are designed in such a way that the coupled pharmaceutical powder cartridges (2, 3) cannot be separated from each other again without damage.

15. Pharmaceutical powder cartridge system according to one of Claims 5 to 14, **characterized in that** the pharmaceutical powder cartridge system (1) also has a device for indicating the quantity of pharmaceutical doses remaining in the storage chambers (4) or removed from the storage chambers (4).

16. Pharmaceutical powder cartridge system according to Claim 15, **characterized in that** the pharmaceutical powder cartridges (2, 3) can be coupled to one another and to the device for indicating the quantity of pharmaceutical doses remaining in the storage chambers (4) or removed from the storage chambers (4).

17. Pharmaceutical powder cartridge system according to either of Claims 15 and 16, **characterized in that** the pharmaceutical powder cartridge system (1) has, for each of the storage chambers (4), a device for indicating the quantity of pharmaceutical doses remaining in the storage chambers (4) or removed from the storage chambers (4).

18. Pharmaceutical powder cartridge system according to one of Claims 1 to 17, **characterized in that** the pharmaceutical powder cartridge system (1) comprises two or more pharmaceutical powder cartridges (2, 3) which, without the locking and indexing means (19, 20, 22, 23, 24), are identical, show mirror-image symmetry or point-symmetry.

19. Pharmaceutical powder cartridge system according to one of the preceding claims, **characterized in that** the metering devices (5) of the individual pharmaceutical powder cartridges (2, 3) have metering cavities (6) of identical or different volume.

20. Inhaler for powdered pharmaceuticals, **characterized in that** the inhaler comprises a pharmaceutical powder cartridge system (1) according to one of the preceding claims.

## Revendications

1. Système (1) de cartouches de poudre de médicament destiné à être utilisé dans un dispositif d'application médicale (inhalateur), à recevoir plusieurs doses d'au moins une substance médicalement active et à être placé de manière remplaçable dans un inhalateur de poudre,
avec au moins deux chambres de réserve séparées l'une de l'autre et présentant la forme de chambres de réserve (4), chacune pour plusieurs doses d'une substance médicalement active,
le système de cartouches de poudre de médicament comprenant au moins une cartouche de poudre de médicament qui présente un dispositif de dosage, le dispositif de dosage présentant pour chacune des chambres de réserve (4) une cavité de dosage (6) qui permet de doser une quantité prédéterminée de chaque substance médicalement active prévue dans les chambres de réserve (4).

2. Système (1) de cartouches de poudre de médicament selon la revendication 1, **caractérisé en ce que** le système (1) de cartouches de poudre de médicament présente en outre un dispositif d'application sensiblement simultanée de doses de médicament provenant d'au moins deux des chambres de réserve séparées l'une de l'autre.

3. Système de cartouches de poudre de médicament selon la revendication 1 ou 2, **caractérisé en ce que** le système de cartouches de poudre de médicament comprend une cartouche de poudre de médicament qui présente au moins deux chambres de réserve (4).

4. Système de cartouches de poudre de médicament selon la revendication 3, **caractérisé en ce que** le système de cartouches de poudre de médicament présente un dispositif qui rassemble les quantités de substances dosées à l'aide des chambres de dosage.

5. Système de cartouches de poudre de médicament selon l'une des revendications 1 ou 2, **caractérisé en ce que** le système (1) de cartouches de poudre de médicament comprend au moins deux cartouches (2, 3) de poudre de médicament qui présentent chacune au moins une chambre de réserve (4).

6. Système de cartouches de poudre de médicament selon la revendication 5, **caractérisé en ce que** chaque cartouche de poudre de médicament (2, 3) présente un dispositif de dosage (5), chaque dispositif de dosage (5) présentant pour chacune des chambres de réserve (4) une cavité de dosage (6) qui permet de doser une quantité prédéterminée de chaque substance médicalement active prévue dans les chambres de réserve (4) des cartouches (2, 3) de poudre de médicament.

7. Système de cartouches de poudre de médicament selon la revendication 6, **caractérisé en ce que** le système (1) de cartouches de poudre de médicament présente un dispositif qui rassemble les quantités de substance dosées à l'aide des chambres de dosage.

8. Système de cartouches de poudre de médicament selon l'une des revendications 6 à 7, **caractérisé en ce que** les dispositifs de dosage (5) peuvent être accouplés.

9. Système de cartouches de poudre de médicament selon l'une des revendications 6 à 8, **caractérisé en ce que** les cartouches (2, 3) de poudre de médicament peuvent être accouplées mécaniquement.

10. Système de cartouches de poudre de médicament selon la revendication 9, **caractérisé en ce que** les cartouches (2, 3) de poudre de médicament présentent en outre sur des surfaces actives (21, 25) qui servent à l'accouplement des cartouches (2, 3) de poudre de médicament des moyens de codage qui permettent d'accoupler uniquement des cartouches (2, 3) de poudre de médicament destinées à cet effet.

11. Système de cartouches de poudre de médicament selon la revendication 10, **caractérisé en ce que** les moyens de codage présentent des saillies et des creux qui permettent uniquement l'accouplement de cartouches (2, 3) de poudre de médicament destinées à cet effet et qui présentent des saillies et des creux accordés mutuellement.

12. Système de cartouches de poudre de médicament selon la revendication 11, **caractérisé en ce que** les saillies et les creux sont disposés en forme de matrice.

13. Système de cartouches de poudre de médicament selon l'une des revendications 5 à 12, **caractérisé en ce que** les cartouches (2, 3) de poudre de médicament présentent en outre sur leurs surfaces actives (21, 25) qui servent à l'accouplement des cartouches (2, 3) de poudre de médicament des moyens d'encliquetage (22, 24, 19, 20, 23) qui permettent l'accouplement des cartouches (2, 3) de poudre de médicament destinées à cet effet.

14. Système de cartouches de poudre de médicament selon la revendication 13, **caractérisé en ce que** les moyens d'encliquetage (22, 24) sont configurés de telle sorte que les cartouches (2, 3) de poudre de médicament accouplées l'une à l'autre ne peuvent être re-séparées l'une de l'autre sans être endommagées.

15. Système de cartouches de poudre de médicament selon l'une des revendications 5 à 14, **caractérisé en ce que** le système (1) de cartouches de poudre de médicament présente en outre un dispositif d'affichage de la quantité de doses de médicament qui reste dans les chambres de réserve (4) ou qui a été prélevée dans les chambres de réserve (4).

16. Système de cartouches de poudre de médicament selon la revendication 15, **caractérisé en ce que** les cartouches (2, 3) de poudre de médicament peuvent être accouplées l'une à l'autre et au dispositif d'affichage de la quantité de doses de médicament qui reste dans les chambres de réserve (4) ou qui a été prélevée dans les chambres de réserve (4).

17. Système de cartouches de poudre de médicament selon l'une des revendications 15 ou 16, **caractérisé en ce que** le système (1) de cartouches de poudre de médicament présente pour chacune des chambres de réserve (4) un dispositif d'affichage de la quantité de doses de médicament qui reste dans les chambres de réserve (4) ou qui a été prélevée dans les chambres de réserve (4).

18. Système de cartouches de poudre de médicament selon l'une des revendications 1 à 17, **caractérisé en ce que** le système (1) de cartouches de poudre de médicament comprend deux ou plusieurs cartouches (2, 3) de poudre de médicament, sans moyens d'encliquetage et de codage (19, 20, 22, 23, 24), identiques, à symétrie spéculaire ou à symétrie ponctuelle.

19. Système de cartouches de poudre de médicament selon l'une des revendications précédentes, **caractérisé en ce que** les dispositifs de dosage (5) des différentes cartouches (2, 3) de poudre de médicament présentent des cavités de dosage (6) qui ont le même volume ou des volumes différents.

20. Inhalateur de médicament poudreux, **caractérisé en ce que** l'inhalateur comprend un système (1) de cartouches de poudre de médicament selon l'une des revendications précédentes.
